Europäisches Patentamt

European Patent Office

Office européen des brevets

(19)

(11) Veröffentlichungsnummer: **0 142 159 B1**

(12) # EUROPÄISCHE PATENTSCHRIFT

(45) Veröffentlichungstag der Patentschrift: **03.07.91**

(51) Int. Cl.⁵: **A61K 31/23, C07C 69/24**

(21) Anmeldenummer: **84113677.3**

(22) Anmeldetag: **13.11.84**

Die Akte enthält technische Angaben, die nach dem Eingang der Anmeldung eingereicht wurden und die nicht in dieser Patentschrift enthalten sind.

(54) **Oberflächenanalgetikum und -antiphlogistikum.**

(30) Priorität: **17.11.83 DE 3341569**
**30.10.84 DE 3439577**

(43) Veröffentlichungstag der Anmeldung:
**22.05.85 Patentblatt 85/21**

(45) Bekanntmachung des Hinweises auf die Patenterteilung:
**03.07.91 Patentblatt 91/27**

(84) Benannte Vertragsstaaten:
**AT BE CH DE FR GB IT LI LU NL SE**

(56) Entgegenhaltungen:
**DE-A- 1 545 304**
**DE-A- 2 251 036**
**FR-A- 2 542 613**

(73) Patentinhaber: **Opel, Helmut, Dr.med.**
**Hudtwalckerstrasse 2-8**
**W-2000 Hamburg 60(DE)**

(72) Erfinder: **Opel, Helmut, Dr.med.**
**Hudtwalckerstrasse 2-8**
**W-2000 Hamburg 60(DE)**

(74) Vertreter: **Siewers, Gescha, Dr.**
**Rechtsanwälte Dr. Harmsen, Dr. Utescher**
**Dipl.-Chem. Harmsen, Bartholatus Dr. Scha-**
**effer, Dr. Fricke, Wolter Patentanwalt Dr. Sie-**
**wers Adenauerallee 28**
**W-2000 Hamburg 1(DE)**

**Beschreibung**

Die Erfindung betrifft die Verwendung flüssiger Fettsäureester oder flüssiger Kohlenwasserstoffe als Oberflächenanalgetika und -antiphlogistika.

Schmerzstillend oder entzündungshemmend wirkende Substanzen zur Verwendung auf Körperoberflächen, insbesondere in der Augenheilkunde, sind in größerer Anzahl bekannt. Die meisten dieser Substanzen sind organische, stickstoffhaltige Verbindungen, die als Basen relativ schlecht wasserlöslich sind und daher bei der Anwendung entweder als freie Basen in öliger Lösung oder in Form ihrer Salze in wässriger Lösung gebraucht werden. Die bisher üblicherweise eingesetzten öligen Lösungsmittel für die freien Basen sind in der Regel sterilisierte pflanzliche Öle, die aber den Nachteil aufweisen, daß sie beispielsweise auf den Schleimhäuten des Auges einen Ölfilm bilden, der von den meisten Patienten als unangenehm angesehen wird. Wässrige Lösungen haben wiederum den Nachteil, daß sie bei häufiger Anwendung das Schleimhautmilieu negativ beeinträchtigen können, da sie zu einer Änderung der osmotischen Bedingungen der Schleimhaut führen.

Es besteht daher immer noch ein Bedürfnis nach einer ölartigen Trägersubstanz für Medikamente für die Anwendung auf Schleimhäuten, die nicht die Nachteile der bisher verwendeten öligen Träger aufweist und insbesondere bei Verwendung in der Augenheilkunde keine Beeinträchtigung des Sehvermögens und unangenehme Sensationen beim Patienten auslöst.

Aufgrund der Überlegung, daß eine ölige Flüssigkeit als Träger dann besonders gut geeignet sein müßte, wenn sie dem menschlichen Hautfett oder verwandten Fetten entspricht oder einen Teil der wesentlichen Inhaltsstoffe dieser Fette aufweist, wurden sogenannte synthetische Bürzeldrüsenöle auf ihre Eignung als Träger bei der Applikation auf Schleimhäuten untersucht. Völlig überraschenderweise wurde festgestellt, daß diese an und für sich bekannten Verbindungen nicht nur eine besondere Eignung als Träger aufweisen, sondern von sich aus auf Schleimhäuten eine analgetische und antiphlogistische Wirkung entfalten.

Die Erfindung betrifft daher die Verwendung dieser sogenannten synthetischen Bürzelöle als Oberflächenanalgetikum und -antiphlogistikum.

Bei der Suche nach Substanzen, die in der Natur vorkommen und in ihrem Wirkungsspektrum Ähnlichkeit mit dem menschlichen Hautfett aufweisen, wurde schon früh das Bürzeldrüsenöl der Wasservögel näher untersucht. Die chemische Analyse am natürlichen Bürzeldrüsenöl zeigte als besonderes Merkmal das Vorhandensein höher Anteile an im wesentlichen vollständig gesättigten verzweigtkettigen Fettsäureestern, deren Gesamtzahl der C-Atome sich im Bereich von etwa 30 bewegt. Das Fett der Bürzeldrüsen ermöglicht es den Vögeln, wenn es in dünner Schicht auf Haut und Federn aufgebracht wird, ihre Körpertemperatur zu halten und ein Benetzen des Gefieders durch Wasser zu verhindern. Diese Eigenschaften haben dazu geführt, daß man entsprechende synthetische Verbindungen schon seit längerem in der Kosmetik als Salbengrundlagen oder Salbenzusätze verwendet, da sich die verzweigtkettigen Fettsäureester durch ein besonderes Spreitvermögen und durch die Abwesenheit einer okklusiven Wirkung auszeichnen. Da die gesunde Haut ständig Wasserdampf und Kohlendioxid an die Umgebung abgibt, führt eine Einschränkung der Wasserverdunstung zu einem zu hohen Wassergehalt und einem Wärmestau; dieses als Okklusion bezeichnete Phänomen bewirkt außerdem eine Steigerung der Keimzahl an der Haut- oder Schleimhautoberfläche und eine qualitative Änderung der Hautflora.

Außerdem war bereits bekannt, daß man die Wirkung des natürlichen Bürzeldrüsenöles nicht nur mit Hilfe verzweigtkettiger Fettsäureester, sondern auch bei Verwendung verzweigtkettiger, im wesentlichen gesättigter Kohlenwasserstoffe erzielen kann. Zu diesen langkettigen gesättigten Kohlenwasserstoffen gehören Beispielsweise Squalan, die gesättigte, dem natürlichen Squalen entsprechende Verbindung oder beispielsweise auch vollsynthetisch hergestellte Verbindungen wie Polyisobutylen mit einer Gesamtkettenlänge von etwa 20 bis 30 C-Atomen oder etwas darüber. Die synthetischen Bürzeldrüsenöle werden bisher in der Kosmetik eingesetzt und seit neuerem auch zur Sanierung ölverschmutzter Wasservögel, da man festgestellt hat, daß die synthetischen Öle nach der Reinigung der verschmutzten Tiere mit Tensiden in der Lage sind, das natürliche Gefiederfett der Wasservögel funktionell zu ersetzen.

Außerdem wurde festgestellt, daß für den angegebenen Verwendungszweck auch synthetische Triglyceride einsetzbar sind, die aus im wesentlichen gesättigten Carbonsäuren mit etwa 8 - 14 C-Atomen in gerader oder verzweigter Kette aufgebaut sind. Derartige Fette sind flüssig und kommen in geringer Menge auch natürlich im Hautfett oder im Bürzelfett vor. Sie werden heute in der Regel synthetisch durch Veresterung von Glyzerin mit Säurefraktionen von Kapryl- bis Myristinsäure und evtl. den entsprechenden verzweigtkettigen Säuren gewonnen. Das Gesamtmolekül hält in der Regel etwa 30 C-Atome, wobei Variationen nach unten und nach oben möglich sind. Gemische derartiger Triglyceride wurden bisher wegen ihres Spreitvermögens in der Kosmetik als Salbengrundlagen verwendet, da sie sich wie auch die beanspruchten Fettsäureester und Kohlenwasserstoffe nicht nur durch ein sehr gutes Spreitvermögen, sondern auch durch das Fehlen einer okklusiven Wirkung auszeichnen.

Die nunmehr bei den Vesuchen des Einsatzes als Träger festgestellte inhärente analgetische und antiphlogistische Wirksamkeit auf Körperoberflächen war völlig überraschend und läßt sich zum jetzigen Zeitpunkt auch noch nicht wissenschaftlich eindeutig erklären, da an und für sich keine analgetisch wirksamen Substanzen bekannt sind, die irgendeine strukturelle Ähnlichkeit aufweisen.

Zwar war aus der DE-OS 15 45 304 bekannt, daß Kohlenwasserstoffgemische oral verabreicht eine analgetische Wirksamkeit aufweisen können. Bei diesen Kohlenwasserstoffgemischen handelt es sich aber um Erölfraktionen, die durch Destillation im Siedebereich zwischen 100 - 140° C gewonnen werden. Dieser Destillationsbereich wird in der Petroleumaufarbeitung Schwerbenzin genannt und enthält nur Kohlenwasserstoffgemische mit 5 - 12 C-Atomen.

Es war daher für den Fachmann überraschend, daß auch völlig andere Kohlenwasserstoffschnitte eine analgetische Wirkung haben können und daß diese außerdem auch bei topischer Anwendung auftritt. Es ist allgemein bekannt, daß fast alle Analgetika, die peroral gut bis ausgezeichnet wirksam sind, bei Verabreichung auf topischem Wege keine oder fast keine Wirksamkeit aufweisen, da die Resorptionsverhältnisse im Magen-Darmtrakt völlig anders als auf Haut oder Schleimhaut sind. Die Tatsache, daß die erfindungsgemäß beanspruchten Verbindungen gerade auf topischem Wege eine gute Wirksamkeit aufweisen, war daher in keiner Weise vorauszusehen.

Bei den sogenannten synthetischen Bürzeldrüsenölen handelt es sich chemisch gesehen entweder um Gemische relativ langkettiger verzweigter Karbonsäuren wie beispielsweise 2-Ethyl-hexansäure mit geradkettigen oder verzweigten Alkoholen mit etwa 16 - 20 C-Atomen oder um im wesentlichen gesättigte verzweigtkettige Kohlenwasserstoffe wie Squalen oder hydrierten Polyisobutylen. Da aber bei der Synthese nicht von reinen Fraktionen ausgegangen wird, liegen in der Regel Gemische mit etwas unterschiedlicher Kettenlänge vor; es können auch sehr geringe Anteile ungesättigter Verbindungen vorhanden sein. Neben den Estern und Kohlenwasserstoffen können auch synthetische Triglyceride eingesetzt werden, die aus im wesentlichen gesättigten Carbonsäuren mit etwa 8 bis 14 C-Atomen in gerader oder vezweigter Kette bestehen. Diese Fette kommen in geringen Mengen auch natürlich im Hautfett oder im Bürzelfett vor. Sie werden heute aber in der Regel synthetisch durch Verästelung von Glycerin durch Säurefraktionen entsprechender Kettenlänge gewonnen.

Die chemisch physikalischen Kennzahlen bewegen sich etwa in folgendem Bereich: Säurezahl unter 1; Verseifungszahl bei den Estern etwa 130 bis 160, bei den Triglyceriden 320 bis 360 und bei den Kohlenwasserstoffen unter 1; Hydroxydzahl unter 1; Biozahl unter 2. Öle dieser Art werden beispielsweise von der Firma BASF unter der Markenbezeichnung "Luvitol" und von der Firma Dragoco unter "PCL/liquid" in den Handel gebracht. Diese synthetischen Bürzeldrüsenöle einschließlich der Triglyceride weisen bei topischer Anwendung auf der Haut oder auf Schleimhäuten eine analgethische und antiphlogistische Wirksamkeit auf. Wichtig bei der Verwendung dieser Verbindungen auf Körperoberflächen ist ihre Spreitwirkung, die mit der Viskosität in Zusammenhang steht. Es wurde festgestellt, daß je nach Applikationsort eine Viskosität zwischen etwa 2 bis 8 mPas in Frage kommen kann, wobei in der Augenheilkunde eine Viskosität um etwa 6 mPas bevorzugt wird.

Unabhängig von der analgethischen und antiphlogistischen Wirksamkeit, aber aufgrund ihrer übrigen Eigenschaften können die erfindungsgemäß eingesetzten Verbindungen nicht nur als solche, sondern auch als Träger für andere Wirkstoffe eingesetzt werden, wobei nicht nur die Aktivität des Wirkstoffes, sondern auch die analgetische und entzündungshemmende Wirkung des Trägers zum Einsatz kommen können. Derartige zusätzliche Wirkstoffe sind beispielsweise die bekannten sonstigen Analgetika, antibakteriell, viruzid oder antimykotisch wirkenden Verbindungen und die üblichen Sympatho- bzw. Parasympathomimetica oder -lytica, die bei der Herstellung der pharmazeutischen Zubereitungen vorzugsweise als Basen oder in Form öllöslicher Salze angesetzt werden.

Im folgenden wird die Erfindung anhand der Beispiele näher erläutert:

Beispiel 1

Patienten mit akuter chemischer oder physikalischer Verletzung der Hornhaut durch Tränengas, metallische Fremdkörper usw. erhielten jeweils einen Tropfen der erfindungsgemäß verwendeten Fettsäureester in das verletzte Auge eingeträufelt. Bereits nach 1 bis 2 Minuten wurde subjektiv von den Patienten Schmerzlinderung oder Schmerzfreiheit angegeben, die durchschnittlich 1 - 4, im Höchstfalle sogar 12 Std. anhielt. Überraschend war auch die Feststellung, daß eine beschleunigte Abheilung ohne Antibiotika oder Cortison erfolgte. Die durchschnittliche Behandlungsdauer betrug 1 bis 5 Tage, je nach Größe und Tiefe der Verletzung.

Beispiel 2

Verschiedenen Patientengruppen mit Hornhautödem, Hornhautulcus und Hornhaut- oder Bindehautnarben nach beispielsweise Herpes Infektionen wurden wie unter Beispiel 1 angegeben, behandelt. Eine entzündungshemmende, antiödematöse Wirksamkeit ließ sich bereits nach einigen Tagen Behandlungszeit feststellen und in allen Fällen erfolgte die Abheilung ohne Komplikationen und ohne zusätzliche Arzneimittelgabe.

Beispiel 3

Patienten mit Tragebeschwerden von weichen oder harten Kontaktlinsen aller gängigen Typen, die z.T. subjektiv schon seit längerem an Schmerzempfindungen litten, wurden wie angegeben behandelt. Auch in diesen Fällen trat die analgetische Wirkung in sehr kurzer Zeit auf und entzündlich beeinflußte Symptome bildeten sich in Kürze zurück.

**Ansprüche**

1. Verwendung flüssiger, im wesentlichen verzweigtikettiger und gesättigter Fettsäurenester oder Kohlenwasserstoffe mit 20 bis 30 C-Atomen im Gesamtmolekül zur Herstellung von Arzneimitteln mit Wirkung als Oberflächenanalgethikum und -antiphlogistikum.

2. Verwendung nach Anspruch 1, dadurch gekennzeichnet, daß die Verbindungen eine Viskosität von 2 bis 8 mPas aufweisen.

3. Verwendung nach Anspruch 1 oder 2, dadurch gekennzeichnet, daß die Verbindungen in Mischung oder Lösung mit weiteren, an sich bekannten Analgetika oder Sympatho oder /Parasympathomimetica oder -lytica vorliegen.

**Claims**

1. Use of liquid mainly branched-chain saturated fatty acid esters or hydrocarbons with 20 to 30 C-atoms in the entire molecule for the production of pharmaceutical preparations acting as surface analgesics and antiphlogistics.

2. Use according to claim 1, characterized in that the compounds have a viscosity of about 2 to 8 mPas.

3. Use according to claim 1 or 2, characterized in that the compounds exist in combination or solution with other actually known analgesics or sympatho- or parasympathomimetics or -lytics.

**Revendications**

1. Utilisation d'esters d'acide gras ou d'hydrocarbures liquides saturés et essentiellement à chaînes ramifiées avec 20 à 30 atomes de carbone dans le molécule entier pour faire des médicaments avec des activités analgésiques et antiphlogistiques.

2. Utilisation selon la revendication 1, caractérisée par le fait que les composés ont une viscosité de 2 à 8 mPas.

3. Utilisation selon les revendications 1 ou 2, caractérisée par le fait que les composés sont présents en mélange ou en solution avec autres analgésiques ou sympatho- ou parasympathomimétiques ou -lytiques déjà connus.